# EUROPEAN PATENT SPECIFICATION

(11) **EP 2 219 520 B1**
(45) Date of publication and mention of the grant of the patent: **12.12.2012**
(21) Application number: 08844185.2
(22) Date of filing: 31.10.2008
(51) Int. Cl.: A61B 5/055, G06T 7/00, G06F 17/50

(54) **METHOD FOR CALCULATING PRESSURES IN A FLUID STREAM THROUGH A TUBE SECTION, ESPECIALLY A BLOOD VESSEL WITH ATHEROSCLEROTIC PLAQUE**
VERFAHREN ZUR BERECHNUNG VON DRÜCKEN IN EINEM FLÜSSIGKEITSSTROM DURCH EINEN RÖHRENFÖRMIGEN ABSCHNITT, INSBESONDERE EIN BLUTGEFÄSS MIT ATHEROSKLEROTISCHEM PLAQUE
PROCEDE DE CALCUL DE PRESSIONS DANS UN COURANT FLUIDE A TRAVERS UNE SECTION TUBULAIRE, NOTAMMENT UN VAISSEAU SANGUIN A PLAQUE ATHEROSCLEROTIQUE

(30) Priority: 31.10.2007 DK 200701555; 31.10.2007 US 1282 P
(43) Date of publication of application: 25.08.2010
(73) Proprietor: CABRA TECHNOLOGY A/S, 9220 Aalborg Øst (DK)
(72) Inventor: KOCK, Samuel Alberg, DK-8220 Brabrand (DK); NYGAARD, Jens Vinge, DK-Hjortshøj (DK); BESENBACHER, Flemming, DK-8210 Århus V (DK); KIM, Won Yong, DK-8000 Århus C (DK)
(74) Representative: Tellefsen, Jens J.
(86) International application number: PCT/DK2008/050266
(87) International publication number: WO 2009/056147

(56) References cited:
- KOCK ET AL.: "Stresses in carotid plaques using MRI-based fluid structure interaction models" PROCEEDINGS OF THE COMSOL USERS CONFERENCE, 24 October 2007 (2007-10-24), XP002514111 Grenoble
- QUARTERONI A ET AL: "Computational vascular fluid dynamics: problems, models and methods" COMPUTING AND VISUALIZATION IN SCIENCE SPRINGER-VERLAG GERMANY, vol. 2, no. 4, March 2000 (2000-03), pages 163-197, XP002514112 ISSN: 1432-9360
- NAZEMI ET AL.: "Pulsatile two-dimensional flow and plaque formation in a carotid artery bifurcation" JOURNAL OF BIOMECHANICS, vol. 23, no. 10, October 1990 (1990-10), XP002514113
- DALIN TANG ET AL: "3D MRI-Based Multicomponent FSI Models for Atherosclerotic Plaques" ANNALS OF BIOMEDICAL ENGINEERING, KLUWER ACADEMIC PUBLISHERS-PLENUM PUBLISHERS, NE, vol. 32, no. 7, 1 July 2004 (2004-07-01), pages 947-960, XP019272821 ISSN: 1573-9686

## Description

### Field of the Invention

The present invention relates to a method for calculating pressures in a fluid stream through a tube section, especially a blood vessel with atherosclerotic plaque.

### Background of the Invention

Atherothrombosis is the leading cause of death and severe disability worldwide (Rosamond et al., 2007). The disease generates fatty deposits inside vessel walls (lipid cores) covered by a protective fibrous cap - the atherosclerotic plaque. If the cap ruptures, blood clots are formed which may be carried downstream to lodge in small-diameter vessels. Disrupted blood flow results in causing heart attacks or strokes. Currently, the risk of cap rupture is assessed using the degree of luminal narrowing. This fails to take the morphology of the plaque into account. Indeed, unstable or vulnerable plaques are known to possess large lipid cores and thin fibrous caps. Morphology such as this generates severe internal stresses in the fibrous cap. In vitro studies have shown that cap rupture predominantly occur when static stresses exceed 300 kPa. The ability to estimate stress and strain magnitudes in the fibrous cap is thus expected to improve risk assessment.

Two large studies, the North American Symptomatic Carotis Endarterectomy Trial (NASCET, 1991) and the European Carotid Surgery Trial (ECST, 1991) have proved carotid endarterectomy to be highly beneficial in symptomatic patients with luminal narrowing of 70% or greater (Rothwell et al., 2003), henceforth designated severe stenosis. The Asymptomatic Carotid Atherosclerosis Study (ACAS, 1995) and the Asymptomatic Carotid Surgery Trial (Halliday et al., 2004) demonstrated that even asymptomatic patients with severe degrees of stenosis may benefit from surgical treatment. Therefore, current risk assessment of carotid atherosclerotic plaques is done by evaluating the degree of luminal stenosis, as measured by conventional intra-arterial angiography, duplex ultrasound, magnetic resonance angiography, or computational tomography.

While increasing degrees of stenosis carries inherent greater risks of atherothrombotic events, most ruptured coronary plaques are less than 60% stenosed (Casscells et al., 2003). As atherosclerosis develops, arteries often dilate in an attempt to normalize elevated wall shear stresses, a process known as expansive remodelling (Glagov et al., 1987). Consequently, risk assessment based purely on the degree of luminal stenosis will tend to underestimate the volume and thereby the severity of the atherosclerotic lesion. Therefore, there is a need for more sensitive methods to risk stratify patients with carotid atherosclerotic disease.

Thrombosis-prone (vulnerable) atherosclerotic plaques have been determined by histological examinations to possess large lipid pools and thin protective fibrous caps (Naghavi et al., 2003). Since plaque rupture, the most frequent cause of thrombosis, by definition represents a structural failure of the protective fibrous cap, it seems reasonable to assume that plaque morphology and biomechanical properties of the atherosclerotic lesion may influence the vulnerability. An in-vitro model study (Li et al., 2006b) showed that thin fibrous caps in combination with large lipid pools generate severe internal fibrous cap stresses. In vitro studies of coronary arteries have shown markedly elevated fibrous cap stresses in ruptured compared to stable lesions (Cheng et al., 1993). A recent publication comparing fibrous cap stress levels in asymptomatic versus symptomatic carotid patients found stresses in symptomatic patients to be nearly twice those of asymptomatic patients (Li et al., 2007).

To estimate stress levels in the fibrous cap, fluid structure interaction (FSI) analysis has emerged as a promising tool combining blood flow simulation through computational fluid dynamics (CFD) with finite element analysis (FEA) of the corresponding stress levels in the surrounding tissues (Tang et al., 2004). Magnetic Resonance Imaging (MRI) is a comprehensive imaging modality for providing geometric models for FSI simulations given its unique in vivo soft tissue imaging capabilities (Saam et al., 2005) combined with the ability to determine blood velocities (Chai and Mohiaddin, 2005) and vessel wall compliance (Metafratzi et al., 2002) non-invasively.

In clinical tests for risk assessment, it is highly desired to have evaluation methods that are fast and reliable. Performing 3D simulations of fibrous cap stresses is very time consuming, and therefore not applicable for rapid characterization of a patient's risk for thrombosis. Therefore, simulations in 2D cross-sections corresponding to either histological data (Cheng et al., 1993) or MRI scans (Li et al., 2006a) have been suggested. Even though the use of cross-sectional data matches the orientation of the available morphologic data, this approach precludes examinations of the longitudinal stress distribution. A previous study (Lovett and Rothwell, 2003) determined carotid plaque ruptures to be symmetrically distributed longitudinally with a majority of ruptures occurring on the upstream side of the plaques. Furthermore, histological examinations of the longitudinal distribution of cell-types indicate differences in smooth muscle cell and macrophage content between upstream and downstream parts of the plaque (Dirksen et al., 1998).

A faster evaluation method has been disclosed in International patent application W02007/002821. The method uses images of cross-sections of the artery or other vessel of interest to identify and locate components of the atherosclerotic deposit, including any hemorrhage, necrotic core, and calcification, and to determine the status and composition of the fibrous cap. A simple scoring system is applied that accounts for the presence of these components and more heavily weights the presence of these components in the juxtaluminal portion of the deposit. This method suffers from the fact that each image has to be evaluated by a person in order to assign values to the degree of occurrence of hemorrhage, necrotic core, and calcification. This human evaluation introduces uncertainties because different persons may judge differently form identical images, which is a great disadvantage.

International patent application WO 2006/069379 discloses a technology to identify structural parameters in order to determine distances, diameters, surface area, volume, etc. This includes the degree of stenosis. However, there is not given any advice as how to calculate pressure variations in the blood vessels for use in simulations of stress distribution around surface irregularities.

In US patent No. 6,377,832 by Bergmann et al. discloses a method for neural networks in a calculation of the severity of a stenosis in a blood vessel.

In International patent application WO 2006/002353 by Chen and Dwyer, a method is disclosed for determining the risk of rupture in a blood vessel using 2-D slice images by scanning a blood vessel and generating a 3-D mesh model of the blood vessel. This method is complicated and requires high computing capacity due to the evaluation of the 3-D model. It would be desirable to provide a simpler model requiring less computing capacity.

NAZEMI ET AL.: "Pulsatile two-dimensional flow and plaque formation in a carotid artery bifurcation" JOURNAL OF BIOMECHANICS, vol. 23, no. 10, October 1990, discloses cross-sectional images of the calculated pressure distribution, but the calculation of the results presented in these images is performed on a 3D model.

QUARTERONI A ET AL: "Computational vascular fluid dynamics: problems, models and methods" COMPUTING AND VISUALIZATION IN SCIENCE SPRINGER-VERLAG GERMANY, vol. 2, no. 4, March 2000, discloses only 2D simulation along the fluid stream.

KOCK ET AL.: "Stresses in carotid plaques using MRI-based fluid structure interaction models" PROCEEDINGS OF THE COMSOL USERS CONFERENCE, 24 October 2007, discloses 3D finite-element simulation of flow and 2D simulation (along the fluid stream) for the fluid-structure interaction problem.

### Object of the Invention

It is the object of the invention to provide a simple, automated method and system for calculation of pressure variations in fluid tubes for use in simulations of longitudinal and radial stress distribution around surface irregularities. Especially, it is the object of the invention to provide specific methods and systems for simulation of stress distribution and blood pressure in blood vessels in order to facilitate predictions of plaque rupture risk.

### Description of the Invention

This purpose is achieved with a method according claim 1.

Particular embodiments are defined in dependent claims 2 to 15.

By the method according to the invention, the pressure distribution in blood vessels can be calculated in a computerised manner. Once having the pressure distribution, methods can be applied for how to simulate stress distribution around surface irregularities. The method is relatively simple and rapid and, therefore, suitable for clinical tests.

In comparison to the aforementioned International Patent Application WO 2006/002353, the method according to the invention is simplified by the 2D sectional image cut following the fluid stream in the tube section with the arms. Thus, the 3-D image is reduced to a 2-D basis, for which calculations are performed fast, simple and reliable even with low computer capacity.

The term cross-sectional is to be understood as a 2D slicing of the tube section across the flow direction in the tube but no necessarily normal to the fluid direction. A certain deviation from a lateral direction, for example a scanning at an angle of between 70° and 89° or between 91 ° and 110° instead of scanning at 90° works equally well and can be taken account of in the calculations.

Though the method is highly suited for simulation of the stress and strain distribution and blood pressure in blood vessels in order to facilitate predictions of plaque cap rupture risk, the method is of general character and may be used for fluid flow in tubes in general, for example water pipes and oil pipes. Especially, it may be used in tubes with material deposits on the inner tube walls.

In an embodiment, the method implies measuring the fluid pressure at the upstream end and measure the fluid velocity at the downstream end and include these measurements in the boundary conditions. For example, the method implies measuring the fluid velocity at the upstream end, and calculating on the basis of the pressure distribution in the tube section the expected fluid velocity at the upstream end, and comparing the measured fluid velocity and the expected fluid velocity at the upstream end as part in an evaluation procedure for the validity of the pressure calculation.

In a further embodiment, the method implies measuring the fluid velocity at the up-stream end and wherein the boundary conditions include the measured fluid velocity at the upstream end and an estimated pressure at the downstream end. For example, the method implies measuring the fluid velocity at the downstream end, and calculating on the basis on the pressure distribution in the tube section the expected fluid velocity at the downstream end, and comparing the measured fluid velocity and the expected fluid velocity at the downstream end as part in an evaluation procedure for the validity of the pressure calculation.

The method implies using the derived longitudinal pressure distribution achieved by either the first or second embodiment as a boundary condition for calculating stresses and deformations in cross-sectional 2D models corresponding to the available image scans, for example MRI scans. Though both the first and second embodiments may be used for longitudinal pressure calculations it would be preferable for the second embodiment to be used to this method's inherent greater accuracy.

The method implies a comparison of the calculated deformations in cross-sectional 2D models with provided cross-sectional scans of the tube section, where the scans depict actual deformations in different cross sectional planes. By comparing the calculated cross sectional deformation with the actual deformation, validation of the chosen tissue parameters can be performed and the accuracy of the simulation can be checked. Preferably, the scans are dynamical scans, however, another possibility is pulsed scans at maximum blood pressure for maximum deformation. In addition, for blood vessels, MRI scans are preferred, however, other scanning methods are also possible, for example ultrasound or X-ray.

As in the case of atherosclerotic plaques in blood vessels or in cases where other kinds of material deposit lumps are found on the inner wall of the tube section obstructing part of a flow path of the fluid in the tube section, the method comprises calculating the forces on the surface of the lump on the basis of the pressure in the tube section at the location of the lump and on the basis of the fluid stream through the section. The calculated forces are then used to perform computer calculations for the deformation of the tube wall due to pressure on the lump. As part in an evaluation procedure for the validity of the applied mechanical parameters this deformation can be correlated with in situ measurements in cross-sectional planes.

As in the case of atherosclerotic plaques in blood vessels, the lump has a cap, and in a further step, the method implies calculating the stress on the cap on the basis of the pressure and the flow of the fluid. The calculated stresses can be assigned to both longitudinal sections and/or cross-sectional planes.

In a practical embodiment, the method comprises the finding of centre points for the fluid stream in the 3D image and calculating a 2D sectional cut through these centre points. Such a cut may not be a straight plane in the case where the tube is bent, as the plane in this case is bent in order to follow the stream. In order to facilitate the calculation, this can be overcome by projecting the image cut onto a straight plane before the calculation of the fluid pressure distribution.

The finding of the centre points may be performed automatically by centroid evaluation functions, however, very good results have been obtained by finding the centre points in the tube section in a manual selection process. In the case that the tube section, for example the blood vessel has a flow divider, which is a usual case, the centre points are found in all arms of the tube section, and a 2D sectional cut is calculated through all centre points.

In the case of blood vessels, the preferred imaging modality is magnetic resonance imaging (MRI), and the method involves measuring the velocity of the fluid in the upstream end or in the downstream end or in both by phase contrast MRI scans. In general the approach applies to any technique capable of acquiring 3D images.

In the following, a semi-automated method of creating longitudinal 2D Fluid Structure Interaction (FSI) models from MRI scans is presented. As shown, this allows simulations of longitudinal stress distributions and blood pressure levels, enables predictions of plaque rupture risk and illustrates the correlations between local stress variation and morphology.

The method according to the invention can also be used for pressure calculation in the heart, especially for evaluating the risk connected to plaque deposits in coronary arteries. In order to image the heart arteries, a method called Virtual Histology can be used, wherein an ultrasonic catheter is scanning the vessel walls from the inside for generating an image of the vessel walls.

It is fundamental to the approach to apply technology capable of separating tissues (morphologies) in the scanned region. The distinction between different cell phenotypes, the extra cellular matrix that cells produce, or the structural regions of different chemical components deposited in the tissue are central in order to generate a computational model of the mechanical stress distribution. The segmentation enables us to assign different properties to distinct regions and hereby model the tissue as a heterogeneous materials rather than a homogeneous. It is the heterogeneity of the structure that allows local peak stress to build up as blood pressure is increased.

### Description of the Drawing

The invention will be explained in more detail with reference to the Drawing with:

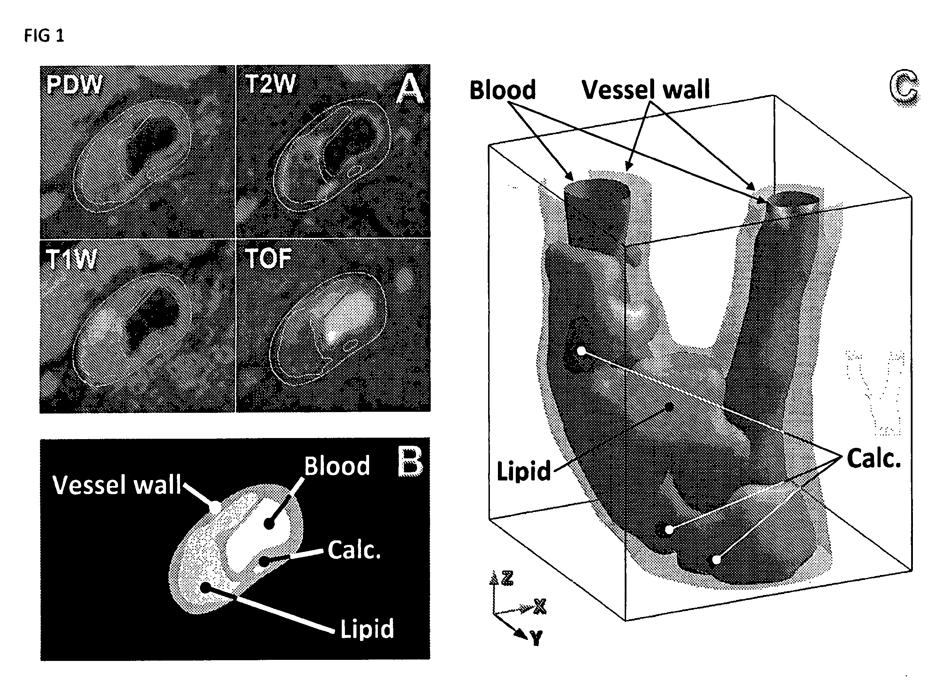

**Figure 1:** (A) Four MRI weightings were performed in order to enable segmentation into blood, vessel wall, and lipid-rich necrotic core. PDW=Proton Density Weighted image, T2W=T2 Weighted image, T1W=T1 Weighted image, TOF=Time Of Flight scan. Isosurfaces (C) surrounding each plaque component were constructed from a collection of grey-scale images (B). Visible features include blood stream, vessel wall, lipid-rich necrotic core, and calcifications.

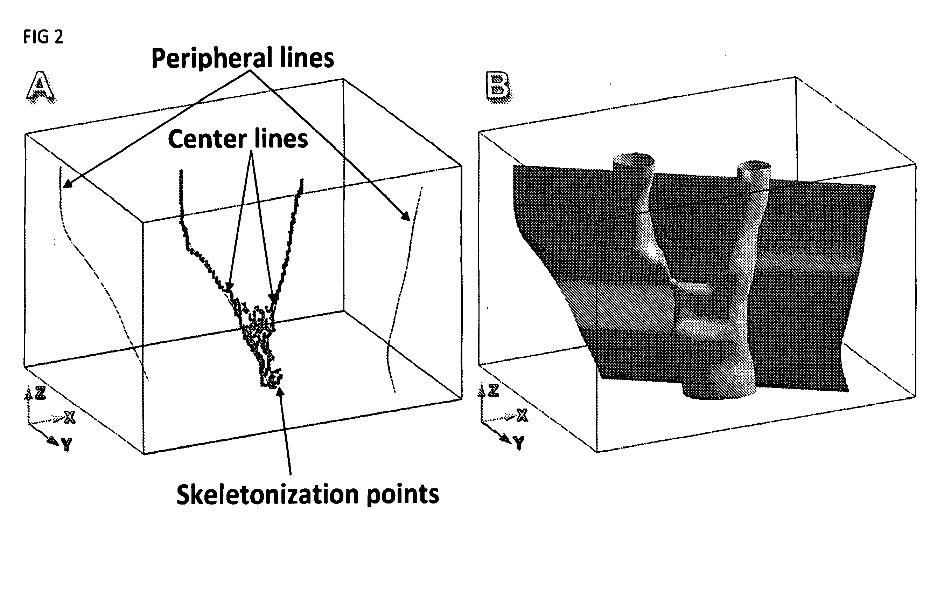

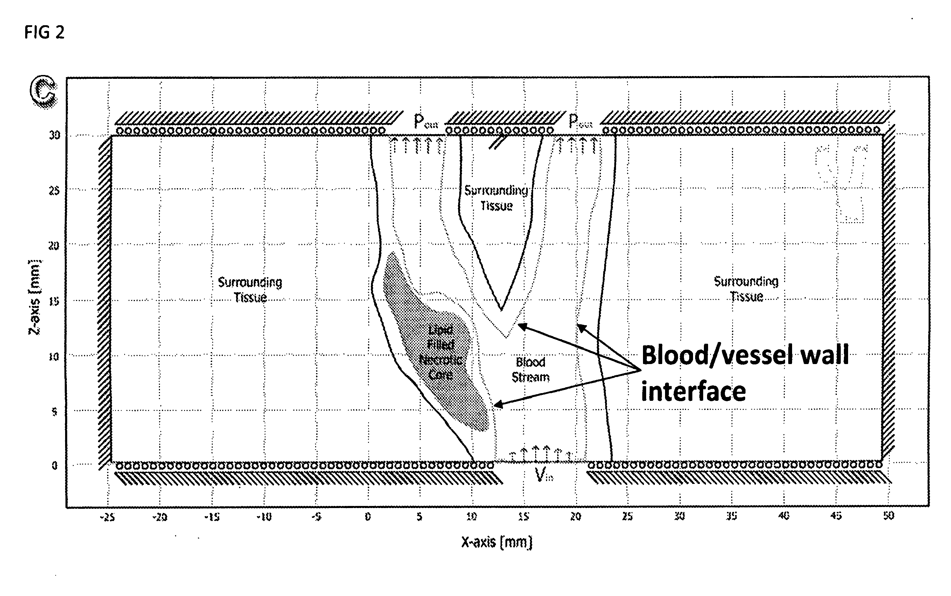

**Figure 2****:** A 3D skeletonization was performed on the blood-stream (A). The internal and external carotids (top) had a nearly circular cross-section yielding unambiguous skeletonization points whilst the common carotid artery (bottom) was ellipsoid in shape resulting in many skeletonization points at this location. Following skeletonization points were manually selected and lines running through the center of the blood streams were created (center lines). These were then extended (peripheral lines) a user-defined distance in order to created a NURBS surface (B) transecting the entire model. The derived longitudinal 2D model (C) was embedded in a slab of surrounding tissue. A parabolic velocity profile was imposed on the bottom inlet (Vin) and outflow pressures were specified at the top outlets as the brachial systolic blood pressure (Pout). Both sides of the model were constrained in all directions; likewise a single point in the top tissue slab between the internal and external carotids was constrained in every direction. Top and bottom sides were allowed to roll horizontally while being constrained vertically. Pressure and viscous forces were coupled from the blood stream to the surrounding tissues through the blood/vessel wall interface.

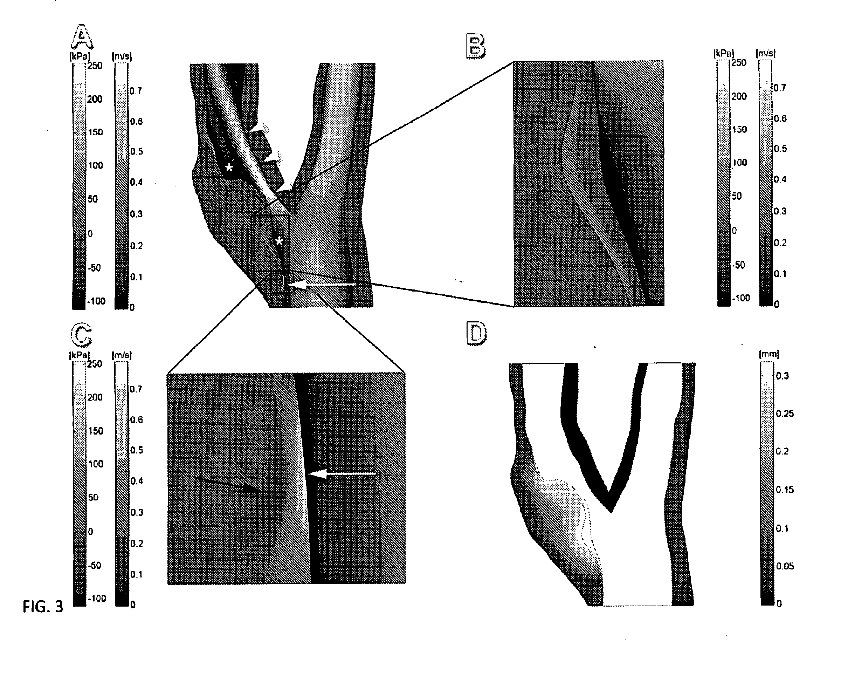

Figure 3: Example of results from an FSI simulation. First principal stresses are presented in the surrounding tissues along with blood velocities (A). The fibrous cap is seen to exhibit severe stresses, maximal at the inlet shoulder (arrow) (see inset C). The severe luminal narrowing resulted in a marked velocity jet (arrowheads) and areas of recirculating blood (asterisks), a known progenitor of further plaque deposition. (D) Deformation of the fibrous cap, vessel wall, and surrounding tissue due to forces imposed by the flowing blood. The deformation can be seen to be maximal above the center of the soft deformable lipid pool deforming towards the periphery. The shoulder regions were located in the transitional zones of high-to-low deformation. Setting mechanic characteristics of the lipid pool to those of normal vessel wall reduced stresses markedly emphasizing the importance of the soft deformable lipid pool with regards to stress levels.

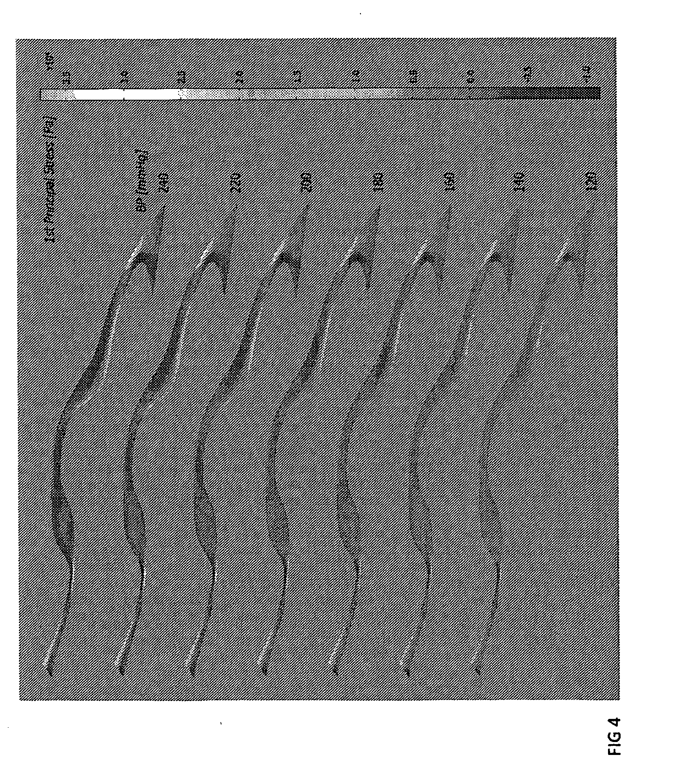

**Figure 4****:** First principal stresses in the fibrous cap as a function of blood pressure. Inflow shoulder region exhibits markedly elevated stress levels as blood pressure increases.

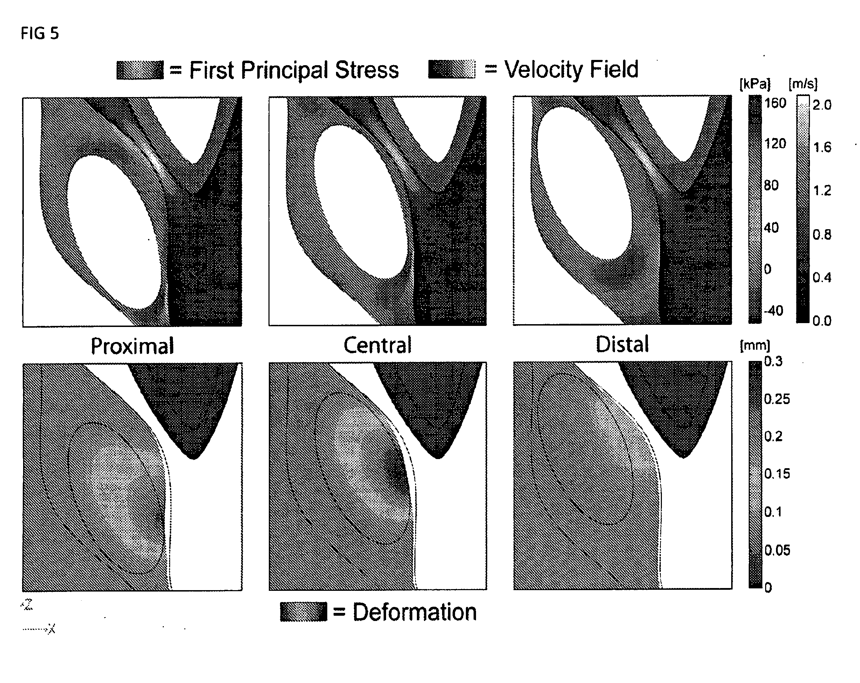

**Figure 5****:** Illustration of the deformation of the vessel and fibrous cap and peak stress calculated for different blood pressure. The inflow shoulder region exhibits markedly elevated stress levels as blood pressure increases. Left image 161.7 kPa, middle image 167.5 kPa, right image 32.3 kPa.

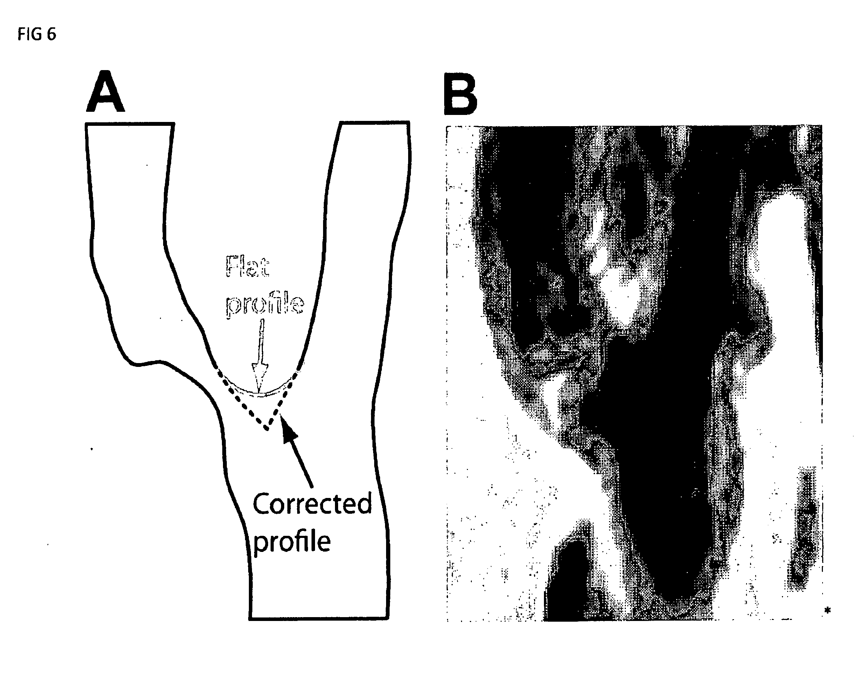

**Figure 6****:** Correction of the flow divider geometry (A) based on a longitudinal MRI scan (B).

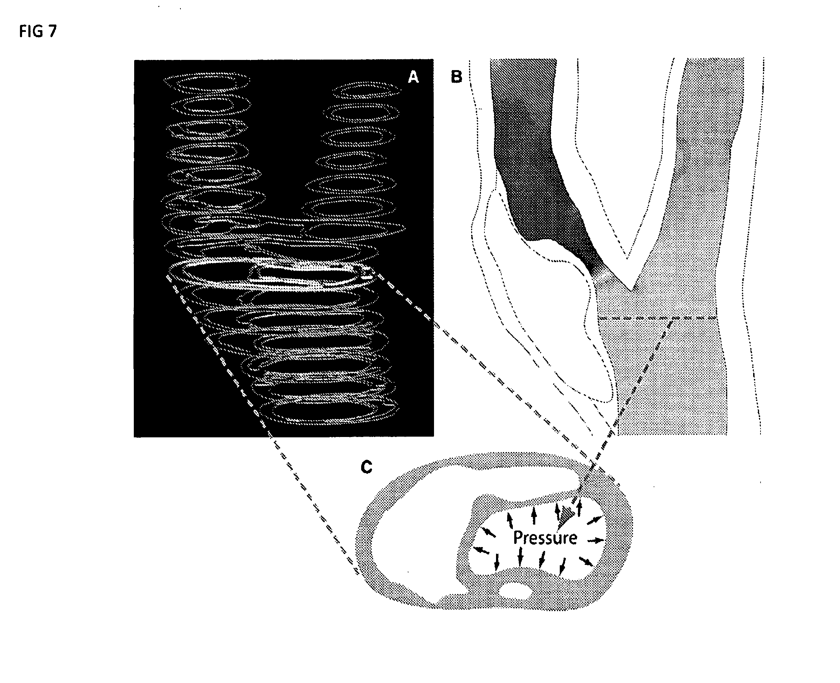

**Figure 7****:** For each of the cross-sectional segmentations (A) also shown in figure 1A and B, a corresponding pressure may be derived from a longitudinal pressure calculation in the fluid (B) (see figure 2C), and used as a boundary condition for cross-sectional 2D models (C) obtained by reusing the data shown in figure 1A and B.

### Detailed Description of the Invention

In the following three different methods will be explained in connection with the invention. The first method is a more simple method, which delivers results superior to prior art methods. However, even better results were obtained by the second method. The third method is applying results from method I or 2 in order to obtain new results in sections coincident with the original 2D scans.

### Method 1

### Magnetic Resonance Imaging

Patients awaiting operation for severe carotid plaque can be scanned by a well-validated MRI protocol (Yuan and Kerwin, 2004) using a Philips 1.5 T scanner (Philips Intera Achieva 1.5T R1.5.4, Philips Inc., Best, The Netherlands). The plaque is scanned using a T1-weighted scan (T1W), T2-weighted scan (T2W), proton-density weighted scan (PDW) and time-of-flight (TOF) scans. Individual plaque components such as lipid, fibrous cap, and calcifications exhibits different image intensities on each scan allowing segmentation approaching histological definitions employed by AHA with a large degree of accuracy(Saam et al., 2005). Sixteen transverse slices centered on the carotid flow divider are acquired using cardiac-gated turbo spin echo sequences: T1W (TR/TE/Inv: 1RR/8/650 ms), T2W and PDW scans with TR=3RR and TE=40/20 ms, and a TOF sequence (TR/TE 34.9/2.4 ms). All sequences cover a field of view of 16x12 cm using a 256x256 matrix resulting in a raw resolution of 0.61x0.61 mm. The final images are interpolated using zero-filled Fourier transforms to a 512x512 matrix and final resolution of 0.3125x0.3125 mm with 2mm slice thickness.

Flow velocities are measured in the transverse plane 2 cm up-and downstream from the flow divider using a phase-contrast turbo field echo sequence (TR/TElVenc: 4.84 ms/2.90 ms/150 cm/s). To resolve the motion of the vessel walls, dynamic balanced steady-state free precession (SSFP) scans with 20 cardiac phases may be performed using a single longitudinal slice and five transverse slices (TR/TE/slice thickness/inplane resolution: 5.7ms/2.8ms/8mm/0.74x0.76mm and 7.7ms/3.9ms/2mm/0.64x0.52mm, respectively). To reduce artifacts stemming from vascular motion all scans should be acquired using ECG-triggering.

### Model generation

The image intensities of each scan compared to the adjacent stemocleid muscle can be analyzed using Cascade, a dedicated semi-automated segmentation tool, allowing segmentation into lipid core, fibrous cap, vessel wall, and blood stream (Liu et al., 2006) (Figure 1A). The components in each slice are exported as a collection of spline curves which are imported into Matlab^{®} (The MathWorks Inc., Natick, MA, USA) and converted to 2D grayscale images (Figure 1B). The images are upscaled to an in-plane resolution of 0.078 mm in order to preserve fine details from the spline curves. From the 2D images a region-of-interest is selected and collected into a single 3D matrix describing the spatial distribution of segmented tissue within the scanned volume. Due to the large difference between the final in-plane resolution of 0.078 mm and slice thickness of 2 mm, the dataset is resampled using linear interpolation and Gaussian smoothing (voxel size = 7, standard deviation = 15). In this way an almost isotropic dataset can be created allowing isosurfaces surrounding each component to be created (Figure 1C).

To create a longitudinal 2D model, the 3D isosurface model is sectioned along a nonuniform rational B-spline (NURBS) surface (Piegl and Tiller, 1997) (Figure 2B) yielding a final 2D model to be analyzed (Figure 2C). The NURBS surface is generated semi-automatically by an initial 3D-skeletonization of the segmented bloodstream. For circular cross-sectional vessel shapes, a single center point will be detected unambiguously while ellipsoid cross-sections will be represented by multiple skeletonization points. Since the carotid vessels may be slightly ellipsoid in shape yielding multiple ambiguous center points, user intervention is suitable in order to select the correct center skeletonization points at either side of the blood stream next to the internal and external carotid arteries (Figure 2A).

In order to create a surface encompassing the entire model, these lines are extended a user-defined distance outwards. All four lines are then used to define a NURBS surface sectioning the central part of the blood vessel throughout the model (Figure 2B). Outlines of the components are created at intersections between the isosurfaces derived from the segmented components and the NURBS surface. These are rotated to lie parallel to the XZ-plane and projected onto this plane. Since the outlines may be curved, geometric approximation errors can be introduced. However, these are considered negligible compared to the smoothing of the initial MRI dataset. To verify the accuracy of the obtained 2D longitudinal model, it is possible to compare the 3D position of the outlines depicted in Figure 2C to the original transverse MRI scans, seen in Figure 1A.

Due to the large slice thickness, the flow divider will often tend to have an unphysiologically flat geometry. To correct for this abnormality, longitudinal MRI scans can be performed (Figure 6B) which include the flow divider position. From these, the flow divider geometry can manually be corrected (Figure 6A). Conceptually simpler model development could be achieved by direct longitudinal scans of the whole section. Unfortunately, it is impossible to assure accurate depiction of both the flow divider position and stenosis morphology. The latter may be too narrow to be included in a single longitudinal scan with a typical thickness of 2 mm.

Though the demonstrated method uses a semi-automated approach with operator intervention, in many cases, an automated method for finding the vessel centre points would be sufficient. This automated method could be performed automatically by a computer program evaluating the density distribution and orientation of the identified centre points.

### Fluid structure interaction model development

From the obtained lines a finite element model was developed employing smoothed splines describing the structural components. From this a coupled fluid-structure interaction (FSI) model was synthesised using COMSOL, a commercially available finite element solver (COMSOL 3.3a, COMSOL Inc, Stockholm, Sweden). The FSI simulation was performed using the FSI plane strain application mode available in the Solid Mechanics module of COMSOL. The FSI model employs a combination of flow simulation in the fluid domain described within the chemical engineering module with the solid mechanics module that simulates the mechanical response of the structure in the solid domain. The coupling is established by obtaining a continuation of the deformation and forces over the boundary that exists between the fluid and the solid domains. Details exists in the COMSOL Multiphysics User's Guide.

### Boundary Conditions

Blood flow is simulated in a Navier-Stokes model, and treated as an incompressible, homogeneous, Newtonian, viscous fluid with a density of 1050 kg/m³ and dynamic viscosity of 0.0035 N·s/m². A parabolic inlet flow profile is applied with the maximal velocity Vᵢₙ measured from the phase-contrast MRI scans (Figure 2C). The two outflows are specified as pressure outlets Pₒᵤₜ identical for both outflows using the brachial blood pressure of the patient. A no-slip boundary condition is applied along the blood-stream/vessel wall interface. The structural components are simulated using a Structural Mechanics plane-strain model. Pressure is used to couple the fluid to the structural deformation along the vessel wall/blood-stream interface. The model is embedded in a rectangle of surrounding tissue with left and right boundaries constrained in both x-and y-directions and top and bottom boundaries (excluding the fluid boundaries) constrained in the y-direction. A single point in the center of the top boundary of the top surrounding tissue block is constrained in both x-and y-directions.

### Material properties

Plaque components are assumed incompressible and to account for the non-linear stress/strain dependency of human tissues, a Neo-Hookean hyper-elastic model is used to specify the material properties of surrounding tissue (µ=6.20e6, κ=1.24e8, ρ=960) and vessel wall (µ=7.20e5, κ=1.44e7, p=1200). Lipid is treated as an isotropic materiel (Tang et al., 2004) with Young's modulus set to 1/100 of that of the equivalent Young's modulus of the vessel wall (E=1e5, v=0.45, p=900).

### Mesh

To reduce computational demands and simulation times, different mesh densities for the individual sub-domains are applied: Vessel wall excluding the fibrous cap and lipid pool are meshed using a maximum element size of 0.3 mm. The blood stream is meshed with a maximum element size of 0.2 mm while the surrounding tissue receives a coarser mesh with a maximum element size of 1 mm. In order to ensure adequate numbers of cells across narrow regions, the fibrous cap separating lipid from blood-flow is meshed with a maximum element size of 0.1 mm.

### Solver

A stationary analysis is employed to simulate the model at the time of maximal inlet velocity. Fluid velocities and corresponding plaque deformation as well as internal principal stresses are calculated and analyzed. To facilitate convergence, artificial isotropic diffusion is applied initially and stepped down logarithmically until finally being removed altogether.

### Results

First principal stress distribution and flow velocities, which are different for the two blood vessel branches, are presented in Figure 3. The results stem from a patient with a high-grade stenosis and substantial lipid core located immediately below the carotid bifurcation beneath the internal carotid artery. The large degree of stenosis gave rise to a marked velocity jet and large areas of recirculating blood, a known progenitor of further plaque deposition. The deformation of the vessel wall, plaque, and surrounding tissue due to forces exerted by the flowing blood is depicted in Figure 3D. The soft lipid pool deformed markedly generating severe stresses in the overlaying fibrous cap, most prominent in the inflow "shoulder region" i.e. the region of the fibrous cap adjacent to the vessel wall. The outflow shoulder region also exhibits elevated first principal stress levels. These areas correspond to the transitional zone from high to low deformation which seems more prone to generation of high stress levels than the severity of luminal stenosis.

Figure 4 depicts first principal stresses caused by blood pressures ranging from 120 to 240 mmHg. The soft lipid pool deformed markedly generating severe stresses (max. 350 kPa) in the overlaying fibrous cap, most prominent in the inflow "shoulder region" i.e. the region of the fibrous cap adjacent to the vessel wall. The outflow shoulder region also exhibits elevated first principal stress levels. A marked jet, visible in the velocity field results from the severe luminal narrowing. Immediately above and below the plaque, large areas of recirculating or slowly moving blood are visible which is a known progenitor of further plaque deposition. Wall expansion is measured via dynamic MRI scans in a cross-section near the inlet. The mean expansion in the present case is 0.9 mm which matches closely with the results of the simulation employing the patient's blood pressure of 160 mmHg.

### Discussion

Despite the indisputable clinical significance of high degrees of stenosis in carotid risk assessment it no longer seems appropriate to base risk stratification solely on this parameter. The invention is a novel solution describing a rapid semi-automated or automated method of determining longitudinal stress levels and blood pressures which can be used in their own right or to improve accuracy of cross-sectional simulations. The technique uses morphologic features derived from in vivo MRI scans and is as such unplagued by geometric problems resulting from histologic preparation or surgical excision.

Results indicate maximal stresses occur in the inflow shoulder region, the preferential site of plaque ulceration (Lovett and Rothwell, 2003). Interestingly, though stresses vary longitudinally, maximal stresses are neither found in the area of maximal constriction, nor in the area with the thinnest fibrous cap. Instead, stress levels were maximal at the transitional zone of high to low deformation. Performing simulations with physical characteristics of the lipid pool set to those of normal vessel wall, i.e. fibrous plaque (Figure 3C), reduces stress levels by an order of magnitude confirming large lipid pools to cause severely elevated stress levels. Our results are thus in agreement with established clinical findings of plaque physiology. Stress levels are calculated using first principal stresses. Though other publications have used Von Mises stresses, we believe first principal stresses are better suited for rupture analyses, since this scale allows depiction of both compressile and tensile forces. It is well-known that human tissues can withstand greater forces when exposed to compression rather than tension making this an important distinction. Though sectioning the model allows rapid simulations to be performed as well as simplifying data analysis the method will inherently disregard morphology out of the section plane. Thus small inclusions of lipid-rich necrotic cores and/or calcifications may be missed with corresponding loss of impact on simulated stress levels. Therefore, the results derived may not accurately depict clinical reality. Since even small changes in vessel diameter may impose severe effects on blood pressure, it was decided that accurate depiction of vessel geometry was of the essence and the NURBS surface was thus designed to intersect the model through the center of the bloodstreams. These problems are inherent whenever 2D simulations are performed, be they longitudinal or cross-sectional; by their very definition, features occurring out of plane will not be included.

The spatial resolutions of the MRI scans leave room for improvement, especially in the longitudinal directions with a slice-thickness of 2 mm. Improvement in this area could facilitate more accurate results. However, enhancing the resolution necessitates prolonged MRI scan times with added costs in terms of scanner time and patient discomfort. Going to higher field strengths (3 tesla or above) may prove beneficial trading off the greater signal to noise ratio (SNR) achieved through higher field strength with the lower SNR from thinner slices. It would have been preferable to use a dynamic solver instead of obtaining a static solution, since dynamic loading is hypothesized to be an additional risk factor. Since blood pressure is used as a boundary condition in the method above, dynamic solving requires knowledge of the blood pressure over time, which we did not have. Noninvasive measurements of dynamic blood pressure are possible using applanation tonometry (Zhao et al., 2002), originally developed for intraocular pressure measurements. It could prove of interest to include dynamically measured blood pressures in future simulations.

### Method 2

In the second method, the parameters were very much alike the first method as described above. However, a number of differences as explained in the following led to an improvement of the method.

Whereas in Method 1, the boundary conditions were
1) identical pressure in the two vessels downstream of the flow divider and
2) measured inlet flow upstream of the flow divider,
and the velocities in the two vessels after the flow divider were estimated on the basis of the numerical results,
the boundary conditions in Method 2 are
1) measured blood velocities in the two vessels downstream of the flow divider and
2) measured pressure upstream of the flow divider,
where the outlet pressures were estimated from the numerical results and the measured inlet velocity upstream of the flow divider used as a control parameter.

In both cases, the blood pressure was calculated around plaque near the flow divider, however, Method 2 yielded more accurate results. Results obtained by Method 2 are illustrated in FIG. 5, where deformation of the vessel and fibrous cap and peak stress has been calculated for different blood pressure. It appears clearly that the inflow shoulder region exhibits markedly elevated stress levels as blood pressure increases.

### Method 3

This method provides stress levels and deformations in 2D model planes coincident with MRI cross-sectional scans and histological sections. By using cross-sectional models, the geometry in the simulations matches the available real tissue morphology as realized by MRI or histology data, facilitating validation of the method by correlations of stress levels and known markers of plaque vulnerability.

Furthermore, whereas in method 1, geometrical errors are introduced due to the Gaussian smoothing when generating the smooth 3D shell models, this is not the case in method 3, since no smoothing needs to be performed when using method 3.

Using either cross-sectional MRI scans or histological data, cross-sectional 2D structural models can be constructed and rapidly simulated, since no flow parameters are calculated using these models. To calculate 2D cross-sectional deformation and stresses, blood pressure is applied along the fluid/solid domain interface, see FIG. 7C. Due to the lack of flow information in the cross-sectional models, assumptions are needed concerning the pressure distribution. Previous simulations have employed uniform longitudinal blood pressure as a boundary condition (Li et al., 2006a). However, it is a fundamental property of a fluid flow in a tube that changes in tube diameter will lead to pressure variations, see Fig. 7B. The assumption of uniform longitudinal blood pressure in a severely stenosed bifurcation is thus problematic. Method 1 and 2 provide a description of the longitudinal pressure variations, which can be sampled at any given cross-sectional plane and applied to a local cross-sectional model as illustrated in figure 7. Though both methods may be used, it would be preferable to combine method 2 and 3, due to the improved pressure specifications of the second method.

### Model development

Method 3 follows the procedures outlined in method 1: model generation. The segmented grayscale images shown in FIG. 1 B are reused to generate a 2D finite element model in any given cross-sectional plane. In this method, simulations of the fluid are not included since no flow is present in the plane of this 2D model. Flow occurs only out of this plane. Hence, the numerical model consists only of the cross-section of the heterogeneous tube. This significantly simplifies the model complexity and reduces simulation time considerably.

### Boundary Conditions

From method 1 and 2 the longitudinal pressure distribution can be derived and an average pressure calculated along horizontal cross-sections or selected from at the surface describing the fluid/solid domain interface, see FIG. 7B. The longitudinal pressure can thus be sampled in a given cross-section and applied as a boundary condition along the vessel wall/blood-stream interface, see FIG. 7C.

The model may be embedded in an artificial domain mimicking the surrounding tissue as depicted by the cross-sectional MRI scans. The boundaries surrounding the centrally located spine are fully constrained with respect to movements leaving the external interface free to deform according to tissue parameters.

### Material properties

A Neo-Hookean hyper-elastic model is used to specify the material properties of surrounding tissue (µ=6.20e6, κ=1.24e8, p=960) and vessel wall (µ=7.20e5, κ=1.44e7, ρ=1200). Lipid is treated as an isotropic materiel (Tang et al., 2004) with Young's modulus set to 1/100 of that of the equivalent Young's modulus of the vessel wall (E=1e5, v=0.45, p=900).

### Mesh

To assure adequate numbers of elements across narrow regions, the resolution of narrow regions is set to 5.

### Solver

The plane strain solid mechanics module in Comsol is used to simulate the stresses and deformation in the cross-sectional models resulting from the applied blood pressure derived from the longitudinal calculations of either method I or 2.

### Validation

The deformation resulting from the mechanical cross-sectional simulations may be compared to dynamic cross-sectional *in vivo* MRI scans depicting the actual deformation in different planes. It is thus possible to validate the chosen tissue parameters and examine the accuracy of the simulation.

### Conclusion

We have presented a novel technique of obtaining rapid longitudinal and radial information of plaque stress levels from 2D transverse MRI scans. The results may be used to investigate longitudinal and radial stress distributions and resulting morphologic and histologic changes as well as provide improved blood pressure boundary specifications for transverse 2D simulations. Initial results are promising conforming to established plaque physiology findings with maximal first principal stress levels found in the inflow region of the fibrous cap approaching established criteria for caps at risk of rupture (Cheng et al., 1993).

### Reference List

ACAS, 1995. Endarterectomy for asymptomatic carotid artery stenosis. Executive Committee for the Asymptomatic Carotid Atherosclerosis Study. Journal of the American Medical Association 273, 1421-1428.
Casscells, W. Naghavi, M. et al., 2003. Vulnerable atherosclerotic plaque: a multifocal disease. Circulation 107, 2072-2075.
Chai, P. Mohiaddin, R., 2005. How we perform cardiovascular magnetic resonance flow assessment using phase-contrast velocity mapping. Journal of Cardiovascular Magnetic Resonance 7, 705-716.
Cheng, G.C. Loree, H.M. et al., 1993. Distribution of circumferential stress in ruptured and stable atherosclerotic lesions. A structural analysis with histopathological correlation. Circulation 87, 1179-1187.
Dirksen, M.T. van der Wal, A.C. et al., 1998. Distribution of inflammatory cells in atherosclerotic plaques relates to the direction of flow. Circulation 98, 2000-2003.
ECST, 1991. MRC European Carotid Surgery Trial: interim results for symptomatic patients with severe (70-99%) or with mild (0-29%) carotid stenosis. European Carotid Surgery Trialists' Collaborative Group. Lancet 337, 1235-1243.
Glagov, S. Weisenberg, E. et al., 1987. Compensatory enlargement of human atherosclerotic coronary arteries. New England Journal of Medicine 316, 1371-1375.
Halliday, A. Mansfield, A. et al., 2004. Prevention of disabling and fatal strokes by successful carotid endarterectomy in patients without recent neurological symptoms: randomised controlled trial. Lancet 363, 1491-1502.
Li, Z.Y. Howarth, S. et al., 2006a. Stress analysis of carotid plaque rupture based on in vivo high resolution MRI. Journal of Biomechanics 39, 2611-2622.
Li, Z.Y. Howarth, S.P.S. et al., 2006b. How Critical Is Fibrous Cap Thickness to Carotid Plaque Stability?: A Flow-Plaque Interaction Model. Stroke 37, 1195-1199.
Li, Z.Y. Howarth, S.P.S. et al., 2007. Structural analysis and magnetic resonance imaging predict plaque vulnerability: A study comparing symptomatic and asymptomatic individuals. Journal of Vascular Surgery 45, 768-775.
Liu, F. Xu, D. et al., 2006. Automated in vivo segmentation of carotid plaque MRI with Morphology-Enhanced probability maps. Magnetic Resonance in Medicine 55, 659-668.
Lovett, J.K. Rothwell, P.M., 2003. Site of carotid plaque ulceration in relation to direction of blood flow: An angiographic and pathological study. Cerebrovascular Diseases 16, 369-375.
Metafratzi, Z.M. Efremidis, S.C. et al., 2002. The clinical significance of aortic compliance and its assessment with magnetic resonance imaging. Journal of Cardiovascular Magnetic Resonance 4, 481-491.
Naghavi, M. Libby, P. et al., 2003. From Vulnerable Plaque to Vulnerable Patient: A Call for New Definitions and Risk Assessment Strategies: Part I. Circulation 108, 1664-1672.
NASCET, 1991. Beneficial effect of carotid endarterectomy in symptomatic patients with high-grade carotid stenosis. North American Symptomatic Carotid Endarterectomy Trial Collaborators. New England Journal of Medicine 325, 445-453.
Piegl, L., Tiller, W., (1997). The NURBS Book. Springer-Verlag, New York, NY.
Rosamond, W. Flegal, K. et al., 2007. Heart Disease and Stroke Statistics--2007 Update: A Report From the American Heart Association Statistics Committee and Stroke Statistics Subcommittee. Circulation 115, e69-171.
Rothwell, P.M. Eliasziw, M. et al., 2003. Analysis of pooled data from the randomised controlled trials of endarterectomy for symptomatic carotid stenosis. Lancet 361, 107-116.
Saam, T. Ferguson, M.S. et al., 2005. Quantitative evaluation of carotid plaque composition by in vivo MRI. Arteriosclerosis, Thrombosis, and Vascular Biology 25, 234-239.
Tang, D. Yang, C. et al., 2004. 3D MRI-based multicomponent FSI models for atherosclerotic plaques. Annals of Biomedical Engineering 32, 947-960.
Yuan, C. Kerwin, W.S., 2004. MRI of atherosclerosis. Journal of Magnetic Resonance Imaging 19, 710-719.
Zhao, S.Z. Ariff, B. et al., 2002. Inter-individual variations in wall shear stress and mechanical stress distributions at the carotid artery bifurcation of healthy humans. Journal of Biomechanics 35, 1367-1377.

## Claims

1. A method for calculating pressures in a fluid stream through a tube section from an upstream end to a downstream end of the tube section, the tube section comprising at least one flow divider dividing the fluid stream from an inlet to at least two arms, each arm with an outlet, the method comprising
- scanning the tube section with a scanner and providing a plurality of cross-sectional 2D scanning images along the fluid stream; said cross-sectional 2D scanning images being a 2D slicing of the section across the flow direction in the tube but not necessarily normal to a fluid direction;
- by a computer program on the basis of the 2D images automatically calculating a 3D image of the tube section by using interpolation between the 2D images,
- by a computer program performing a 2D sectional image cut through the 3D image, the 2D sectional image cut following the fluid stream in the tube section with the arms,
- calculating in the 2D sectional image cut a fluid pressure distribution along the tube section on the basis of given boundary conditions, the boundary conditions including fluid velocity or fluid pressure at the upstream end;
**characterised in that** using said fluid pressure distribution as a boundary condition for
- calculating stresses and deformations of the tube section in cross-sectional 2D models; said cross-sectional 2D models corresponding to said 2D scanning images,
- providing cross-sectional scans of the tube section depicting the actual deformation in different cross sectional planes for
- comparing the calculated cross sectional deformation with the actual deformation for validating the chosen tissue parameters and examining the accuracy of the simulation.

2. The method according to claim 1, wherein the method implies measuring the fluid pressure at the upstream end, measuring the fluid velocity at the downstream end and including these measurements in the boundary conditions.

3. The method according to claim 2, wherein the method implies measuring the fluid velocity at the upstream end, calculating the expected fluid velocity at the upstream end on the basis on the pressure distribution in the tube section, and comparing the measured fluid velocity and the expected fluid velocity at the upstream end as part of an evaluation procedure for the validity of the pressure calculation.

4. The method according to claim 1, wherein the method implies measuring the fluid velocity at the upstream end and wherein the boundary conditions include the measured fluid velocity at the upstream end and an estimated pressure at the downstream end.

5. The method according to claim 4, wherein the method implies measuring the fluid velocity at the downstream end, calculating the expected fluid velocity at the downstream end on the basis on the pressure distribution in the tube section, and comparing the measured fluid velocity and the expected fluid velocity at the downstream end as part of an evaluation procedure for the validity of the pressure calculation.

6. The method according to any preceding claim, wherein the tube section comprises a material deposit lump on the inner wall of the tube section obstructing part of a flow path of the fluid in the tube section, and wherein the method comprises calculating the forces on the surface of the lump on the basis of the pressure in the tube section at the location of the lump.

7. The method according to claim 6, wherein the tube section has an elastic wall and wherein the method implies performing computer calculations for the deformation of wall due to pressure on the lump.

8. The method according to claim 7, wherein the lump has a cap, and wherein the method implies calculating the stress on the cap on the basis of the pressure and the flow of the fluid.

9. The method according to any preceding claim, wherein the method comprises finding centre points for the fluid stream in the 3D image and calculating a 2D sectional cut through these centre points.

10. The method according to claim 9, wherein the method implies projecting the image cut onto a straight plane before the calculation of the fluid pressure distribution.

11. The method according to claim 9 or 10, wherein the finding of the centre points involves manual selecting the centre points in the tube section, the method involving finding the centre points in all arms of the tube section, if the tube section has more than one arm, and calculating a 2D sectional cut through all centre points.

12. The method according to any preceding claim, wherein the scanner is an MRI scanner, and wherein the method involves measuring the velocity of the fluid in the upstream end or in the downstream end or in both by phase contrast MRI scans.

13. The method according to any preceding claim, wherein the fluid is blood, the tube section is part of a blood vessel and the lump comprises atherosclerotic plaques with a thin protective fibrous cap.

14. The method according to claim 1, wherein the method comprises using said fluid pressure distribution for calculating an average pressure along fluid/solid domain interfaces for calculating 2D cross-sectional deformation and stresses of the tube and of material deposits in the tube, if present; said fluid/solid domain interfaces defining an interface between an inner wall of the tube and a fluid in the tube or an interface between a lump of material arranged on the inner wall of the tube and the fluid in the tube.

15. The method according to claim 14, the method further comprising comparing the calculated cross sectional deformation to dynamic cross-sectional MRI scans depicting the actual deformation in different cross sectional planes for validating the chosen tissue parameters and examining the accuracy of the simulation.

## Patentansprüche

1. Verfahren zum Berechnen von Druck in einem Fluidstrom durch einen Rohrausschnitt von einem stromaufwärtigen Ende bis zu einem stromabwärtigen Ende des Rohrausschnittes, wobei der Rohrausschnitt wenigstens einen den Fluidstrom von einem Einlauf in wenigstens zwei Zweige teilenden Mengenteiler aufweist, wobei jeder Zweig einen Auslauf aufweist, wobei das Verfahren folgende Verfahrensschritte umfasst:
- Abtasten des Rohrausschnittes mit einem Abtaster und Erzeugen einer Vielzahl von querschnittlichen 2D Abtastaufnahmen entlang dem Fluidstrom; die querschnittlichen 2D-Abtastaufnahmen sind 2D-Schnitte vom Schnitt quer zur Stromrichtung im Rohr, aber nicht unbedingt senkrecht zu einer Fluidrichtung;
- durch ein Computerprogramm anhand von den 2D-Aufnahmen ein selbsttätiges Berechnen eines 3D-Bildes von dem Rohrausschnitt durch Interpolation zwischen den 2D-Aufnahmen,
- durch ein Computerprogramm Ausführung eines durch das 3D-Bild geschnittenen 2D-Bildes, wobei das 2D-Schnittbild dem Fluidstrom im Rohrausschnitt mit den Zweigen folgt,
- Berechnen einer Fluiddruckverteilung in dem 2D-Schnittbild entlang dem Rohrausschnitt anhand von den gegebenen Randbedingungen, wobei die Randbedingungen auch die Fluidgeschwindigkeit oder Fluiddruck am stromaufwärtigen Ende umfassen;
**dadurch gekennzeichnet, dass**
die Fluiddruckverteilung verwendet wird als Randbedingung für
- das Berechnen von Spannungen und Verformungen des Rohrausschnittes in querschnittlichen 2D-Modellen; die erwähnten querschnittlichen 2D-Modellen entsprechen den erwähnten 2D-Abtastaufnahmen,
- Bereitstellen querschnittlicher Scans vom Rohrausschnitt als Darstellung der tatsächlichen Verformung in verschiedenen Querschnittsebenen zum
- Vergleichen der berechneten querschnittlichen Verformung mit der tatsächlichen Verformung zur Validierung der gewählten Gewebeparameter und Untersuchung der Simulationsgenauigkeit.

2. Verfahren nach Anspruch 1, wobei das Verfahren das Messen des Fluiddruckes am stromaufwärtigen Ende, das Messen der Fluidgeschwindigkeit am stromabwärtigen Ende und das Einbeziehen dieser Messungen in die Randbedingungen impliziert.

3. Verfahren nach Anspruch 2, wobei das Verfahren das Messen der Fluidgeschwindigkeit am stromaufwärtigen Ende, Berechnen der erwarteten Fluidgeschwindigkeit am stromaufwärtigen Ende anhand von der Druckverteilung im Rohrausschnitt, und Vergleichen der gemessenen Fluidgeschwindigkeit mit der erwarteten Fluidgeschwindigkeit am stromaufwärtigen Ende als Teil eines Auswertungsverfahrens für die Gültigkeit der Druckberechnung impliziert.

4. Verfahren nach Anspruch 1, wobei das Verfahren das Messen der Fluidgeschwindigkeit am stromaufwärtigen Ende impliziert, und wobei die Randbedingungen die gemessene Fluidgeschwindigkeit am stromaufwärtigen Ende und einen veranschlagten Druck am stromabwärtigen Ende umfassen.

5. Verfahren nach Anspruch 4, wobei das Verfahren das Messen der Fluidgeschwindigkeit am stromabwärtigen Ende, das Berechnen der erwarteten Fluidgeschwindigkeit am stromabwärtigen Ende anhand von der Druckverteilung im Rohrausschnitt, sowie das Vergleichen der gemessenen Fluidgeschwindigkeit mit dem erwarteten Fluidgeschwindigkeit am stromabwärtigen Ende als Teil eines Auswertungsverfahrens für die Gültigkeit der Druckberechnung impliziert.

6. Verfahren nach irgendeinem der vorhergehenden Ansprüche, wobei der Rohrausschnitt einen Materialkloß am Innenwand des Rohrausschnittes aufweist, der einen Teil des Fluidstromweges im Rohrausschnitt blockiert, und wobei das Verfahren das Berechnen von Kräften an die Kloßoberfläche anhand von dem Druck im Rohrausschnitt an der Stelle des Kloßes umfasst.

7. Verfahren nach Anspruch 6, wobei der Rohrausschnitt eine elastische Wand aufweist, und wobei das Verfahren Computerberechnungen der Verformung der Wand wegen Druck auf den Kloß impliziert.

8. Verfahren nach Anspruch 7, wobei der Kloß eine Kappe aufweist, und wobei das Verfahren das Berechnen der Spannung auf die Kappe anhand von dem Druck und dem Strom des Fluids impliziert.

9. Verfahren nach irgendeinem der vorgehenden Ansprüche, wobei das Verfahren das Feststellen von Mittelpunkten des Fluidstroms im 3D-Bild und das Berechnen von einem 2D-Schnitt durch diese Mittelpunkte umfasst.

10. Verfahren nach Anspruch 9, wobei das Verfahren die Projektion vom Schnittbild auf eine gerade Fläche vor das Berechnen der Fluiddruckverteilung impliziert.

11. Verfahren nach Anspruch 9 oder 10, wobei das Feststellen der Mittelpunkte ein manuelles Auswählen von Mittelpunkten im Rohrausschnitt umfasst, wobei das Verfahren das Feststellen von Mittelpunkten in allen Zweigen des Rohrausschnittes, wenn der Rohrausschnitt mehr als einen Zweig aufweist, und Berechnen eines 2D-Schnittes durch alle Mittelpunkte umfasst.

12. Verfahren nach irgendeinem der vorgehenden Ansprüche, wobei der Abtaster ein MRT-Abtaster ist, und wobei das Verfahren das Messen der Fluidgeschwindigkeit im stromaufwärtigen Ende oder im stromabwärtigen Ende oder beiden durch Phasenkontrast-MRT-Scans umfasst.

13. Verfahren nach irgendeinem der vorhergehenden Ansprüche, wobei das Fluid Blut ist, der Rohrausschnitt ein Teil eines Blutgefässes ist, und der Kloß atherosclerotische Beläge mit dünnen, faserigen Schutzkappen umfasst.

14. Verfahren nach Anspruch 1, wobei das Verfahren die Verwendung der erwähnten ersten Fluiddruckverteilung zum Berechnen eines durchschnittlichen Druckes entlang den Fluid/Feststoff-Domäne-Grenzflächen zum Berechnen der 2D querschnittlichen Verformung und Spannungen des Rohrs und eventueller Materialklöße im Rohr umfasst; wobei die Fluid/Feststoff-Domäne-Grenzflächen eine Grenzfläche zwischen einer Rohrinnenwand und einer Flüssigkeit im Rohr oder eine Grenzfläche zwischen einem Materialkloß am Rohrinnenwand und dem Fluid im Rohr definieren.

15. Verfahren nach Anspruch 14, wobei das Verfahren ferner das Vergleichen der berechneten querschnittlichen Verformung mit den dynamischen querschnittlichen, die tatsächliche Verformung in verschiedenen Querschnittsebenen darstellenden MRT-Scans zur Validierung der gewählten Gewebeparameter und Untersuchung der Simulationsgenauigkeit umfasst.

## Revendications

1. Procédé de calcul de pressions dans un courant fluide à travers une section tubulaire allant d'une extrémité amont à une extrémité aval de la section tubulaire, la section de tube comprenant au moins un séparateur de flux séparant le courant fluide d'une entrée vers au moins deux bras, chaque bras pourvu d'une sortie, le procédé comprenant
- de scanner la section tubulaire avec un scanner et d'obtenir une pluralité d'images de scannage 2D en coupe transversale le long du courant fluide; lesdits images de scannage 2D en section transversale constituant un découpage 2D de la section à travers la direction d'écoulement dans le tube mais pas nécessairement normal pour la direction du fluide;
- par un programme d'ordinateur sur la base des images 2D de calculer automatiquement une image tridimensionnelle de la section tubulaire par interpolation entre les images 2D,
- par un programme d'ordinateur de mettre en oeuvre une image en coupe 2D coupée à travers l'image 3D, l'image en coupe 2D coupé suivant le flux de fluide dans la section tubulaire avec les bras
- de calculer dans l'image en coupe 2D coupé une répartition de la pression d'un fluide le long de la section tubulaire sur la base des conditions limites données, les conditions limites comprenant la vitesse du fluide ou la pression du fluide au niveau de l'extrémité amont; **caractérisé en ce que**
l'utilisation de ladite répartition du fluide de pression en tant qu'une condition limite pour
- calculer des contraintes et des déformations de la section tubulaire dans des modèles 2D en coupe transversale; lesdits modèles 2D en coupe transversale correspondant auxdites images de scannage 2D,
- fournir des scannages transversales de la section tubulaire décrivant la déformation réelle dans des différents plans de section transversale pour
- la comparaison de la déformation transversale calculée avec la déformation réelle pour valider les paramètres de tissu sélectionné et examiner la précision de la simulation.

2. Procédé selon la revendication 1, où le procédé consiste à mesurer la pression du fluide au niveau de l'extrémité amont, à mesurer la vitesse du fluide au niveau de l'extrémité aval et à comprendre ces mesurages dans les conditions limites.

3. Procédé selon la revendication 2, où le procédé consiste à mesurer la vitesse du fluide au niveau de l'extrémité amont, à calculer la vitesse de fluide prévue au niveau de l'extrémité en amont sur la base de la répartition de la pression dans la section tubulaire, et à comparer la vitesse du fluide mesurée et la vitesse de fluide prévue au niveau de l'extrémité amont en tant que partie d'une procédure d'évaluation de la validité du calcul de pression.

4. Procédé selon la revendication 1, où le procédé consiste à mesurer la vitesse du fluide au niveau de l'extrémité amont et où les conditions limites comprennnent la vitesse du fluide mesurée au niveau de l'extrémité en amont et une pression prévue au niveau de l'extrémité aval.

5. Procédé selon la revendication 4, où le procédé consiste à mesurer la vitesse du fluide au niveau de l'extrémité aval, à calculer la vitesse de fluide prévue au niveau de l'extrémité aval sur la base de la répartition de la pression dans la section tubulaire, et à comparer la vitesse du fluide mesurée et la vitesse de fluide prévue au niveau de l'extrémité aval en tant que partie d'une procédure d'évaluation de la validité du calcul de pression.

6. Procédé selon l'une quelconque des revendications précédentes, où la section tubulaire comprend un noeud de dépôt de matière sur la paroi intérieure de la section tubulaire faisant obstacle à une partie d'un filet de fluide dans la section tubulaire, et où le procédé consiste à calculer les forces exercées sur la surface du noeud en fonction de la pression dans la section tubulaire au niveau de l'emplacement du noeud.

7. Procédé selon la revendication 6, où la section tubulaire comporte une paroi élastique et où le procédé consiste à effectuer des calculs informatiques permettant la déformation de la paroi du fait de la pression sur le noeud.

8. Procédé selon la revendication 7, où le noeud possède un capuchon, et où le procédé consiste à calculer la contrainte sur le capuchon sur la base de la pression et de l'écoulement du fluide.

9. Procédé selon l'une quelconque des revendications précédentes, où le procédé consiste à trouver des points centraux pour le courant de fluide dans l'image 3D et à calculer une coupe de section 2D à travers ces points centraux.

10. Procédé selon la revendication 9, où le procédé consiste à projeter l'image coupée sur un plan droit avant le calcul de la distribution de pression de fluide.

11. Procédé selon la revendication 9 ou 10, où la recherche des points centraux implique la sélection manuelle des points centraux de la section tubulaire, le procédé consistant à trouver les points centraux dans tous les bras de la section tubulaire, si la section tubulaire présente plus qu'un bras, et le calcul d'une coupe de section 2D à travers tous les points centraux.

12. Procédé selon l'une quelconque des revendications précédentes, où le scanner est un scanner IRM, et où le procédé consiste à mesurer la vitesse du fluide dans l'extrémité amont ou dans l'extrémité en aval ou dans les deux, par scannage IRM à contraste de phase.

13. Procédé selon l'une quelconque des revendications précédentes, où le fluide est du sang, la section tubulaire est une partie d'un vaisseau sanguin et le noeud comprend des plaques d'athérosclérose avec un capuchon fibreux de protection mince.

14. Procédé selon la revendication 1, où le procédé comprend l'utilisation de ce fluide de la répartition de la pression pour calculer une pression moyenne le long des interfaces du domaine fluide/solide pour le calcul de la déformation 2D transversale et les contraintes du tube et des dépôts de matière dans le tube, s'il est présent; lesdites interfaces du domaine fluide/solide définissant une interface entre une paroi intérieure du tube et un fluide dans le tube ou une interface entre un morceau de matière disposé sur la paroi intérieure du tube et le fluide dans le tube.

15. Procédé selon la revendication 14, le procédé comprenant en outre la comparaison de la déformation de section transversale calculée avec les scannages IRM dynamiques transversales représentant la déformation réelle dans différents plans de section transversale pour valider les paramètres de tissus choisis et examiner la précision de la simulation.
